# EUROPEAN PATENT SPECIFICATION

(11) **EP 4 146 063 B1**
(45) Date of publication and mention of the grant of the patent: **10.09.2025**
(21) Application number: 21800249.1
(22) Date of filing: 06.01.2021
(51) Int. Cl.: A61B 5/0245, A61B 5/251, A61B 5/28, A61B 5/308, A61B 5/318, A61B 5/346, A61B 5/349, A61F 2/24, A61N 1/365

(54) **PROSTHETIC AORTIC VALVE PACING SYSTEMS**
PROTHETISCHE AORTENKLAPPEN-SCHRITTMACHERSYSTEME
SYSTÈMES DE STIMULATION DE VALVULE AORTIQUE PROTHÉTIQUE

(30) Priority: 06.05.2020 US 202016868121
(43) Date of publication of application: 15.03.2023
(73) Proprietor: E-Valve Systems Ltd., Herzliya 4600843 (IL)
(72) Inventor: GROSS, Yossi, 73160 Moshav Mazor (IL)
(74) Representative: Evens, Paul Jonathan
(86) International application number: PCT/IL2021/050017
(87) International publication number: WO 2021/224904

(56) References cited:
- US-A- 5 487 760
- US-A1- 2006 178 707
- US-A1- 2010 100 144
- US-A1- 2014 371 841
- US-A1- 2017 258 585
- US-A1- 2017 266 433
- US-A1- 2019 209 302
- US-A1- 2019 314 150
- US-A1- 2020 261 224
- US-B2- 7 643 879

## Description

### CROSS-REFERENCE TO RELATED APPLICATIONS

The present application claims priority from US Application 16/868,121, filed May 6, 2020, which is a continuation-in-part of US Application 16/734,798, filed January 6, 2020, now U.S. Pat. No. 10,835,750, which (a) is a continuation-in-part of US Application 15/864,661, filed January 8, 2018, now U.S. Pat. No. 10,543,083, and (b) claims foreign priority to European Application 19150581.7, filed January 7, 2019, which published as EP 3 508 113 A1. All of the above-referenced applications are assigned to the assignee of the present application.

(The above-mentioned European Application 19150581.7 claims foreign priority to the above-mentioned US Application 15/864,661.)

### FIELD OF THE APPLICATION

The present invention relates generally to surgical implants and systems, and specifically to a valve prosthesis system.

### BACKGROUND OF THE APPLICATION

Aortic heart valve replacement may be necessary to treat valve regurgitation or stenotic calcification of the leaflets. In percutaneous transluminal delivery techniques, a prosthetic aortic valve is compressed for delivery in a catheter and advanced through the descending aorta to the heart, where the prosthetic valve is deployed in the aortic valve annulus. New-onset cardiac conduction disturbances are common after transcatheter aortic valve implantation (TAVI). The most common complication is left bundle branch block (LBBB).

US Patent 7,914,569 to Nguyen et al. describes a heart valve prosthesis having a self-expanding multi-level frame that supports a valve body comprising a skirt and plurality of coapting leaflets. The frame transitions between a contracted delivery configuration that enables percutaneous transluminal delivery, and an expanded deployed configuration having an asymmetric hourglass shape. The valve body skirt and leaflets are constructed so that the center of coaptation may be selected to reduce horizontal forces applied to the commissures of the valve, and to efficiently distribute and transmit forces along the leaflets and to the frame. Alternatively, the valve body may be used as a surgically implantable replacement valve prosthesis.

US 2017/266433 relates to a pacing guidewire. US 7643879 relates to an integrated cardia rhythm management system with heart valve. US 2019/314150 relates to an artificial valve.

### SUMMARY OF THE APPLICATION

In accordance with the present invention, there is provided a valve prosthesis system as defined in appended independent claim 1. Embodiments of the present invention are defined in the appended claims dependent on independent claim 1.

Some arrangements of the present disclosure provide a valve prosthesis system, which comprises a prosthetic aortic valve, which is configured to be implanted in a native aortic valve of a patient, and which comprises a plurality of prosthetic leaflets, a frame, and one or more electrodes, including a cathode and an anode, mechanically coupled to the frame. The prosthetic aortic valve further comprises a prosthetic-valve coil, which is in non-wireless electrical communication with the cathode and the anode. In embodiments of the present invention, the prosthetic aortic valve does not comprise any active electronic components.

In embodiments of the present invention, the valve prosthesis system further comprises a non-implantable unit, which comprises an energy-transmission coil; at least two sensing skin ECG electrodes; and non-implantable control circuitry. The non-implantable control circuitry is configured to:
- drive the cathode and the anode to apply a pacing signal to a heart of the patient,
- detect at least one cardiac parameter using the at least two sensing skin ECG electrodes, and
- at least partially responsively to the detected at least one cardiac parameter, set parameters of the pacing signal, by wirelessly transferring energy from the energy-transmission coil to the prosthetic-valve coil by inductive coupling.

For some applications, the frame is shaped so as to define an upstream inflow portion; a downstream outflow portion; and a constriction portion, which is axially between the upstream inflow portion and the downstream outflow portion. The prosthetic leaflets are coupled to the constriction portion.

For some applications, when the prosthetic aortic valve is in an expanded fully-deployed configuration: free edges of the prosthetic leaflets face toward the downstream outflow portion, and a ring-shaped longitudinal border between the downstream outflow portion and the constriction portion is defined by a downstream-most point of the frame to which the prosthetic leaflets are coupled. The prosthetic aortic valve further comprises a prosthetic-valve coil, which is in non-wireless electrical communication with the one or more electrodes, and which is coupled to the frame no more than 1 mm upstream of the ring-shaped longitudinal border, such as axially along the downstream outflow portion.

In some arrangements of the present disclosure, a valve prosthesis system is provided, which includes a prosthetic aortic valve and a non-implantable unit. The prosthetic aortic valve which includes a plurality of prosthetic leaflets; a frame; a cathode and an anode, which are mechanically coupled to the frame; and a prosthetic-valve coil, which is in non-wireless electrical communication with the cathode and the anode. The non-implantable unit includes an energy-transmission coil; and non-implantable control circuitry, which is configured to drive the cathode and the anode to apply a pacing signal and to set parameters of the pacing signal, by wirelessly transferring energy from the energy-transmission coil to the prosthetic-valve coil by inductive coupling.

There is therefore provided, in accordance with the present invention, a valve prosthesis system including:
(i) a prosthetic aortic valve, which is configured to be implanted in a native aortic valve of a patient, and which includes:
   (a) a plurality of prosthetic leaflets;
   (b) a frame;
   (c) a cathode and an anode, which are mechanically coupled to the frame; and
   (d) a prosthetic-valve coil, which is in non-wireless electrical communication with the cathode and the anode, wherein the prosthetic aortic valve does not include any active electronic components; and
(ii) a non-implantable unit, which includes:
   (a) an energy-transmission coil;
   (b) at least two sensing skin ECG electrodes; and
   (c) non-implantable control circuitry, which is configured to:
      drive the cathode and the anode to apply a pacing signal to a heart of the patient,
      detect at least one cardiac parameter using the at least two sensing skin ECG electrodes, and
      at least partially responsively to the detected at least one cardiac parameter, set parameters of the pacing signal, by wirelessly transferring energy from the energy-transmission coil to the prosthetic-valve coil by inductive coupling.

In the he valve prosthesis system, the non-implantable control circuitry may be configured to:
analyze the detected at least one cardiac parameter to assess a level of responsiveness of the heart to the pacing signal, and
upon ascertaining that the level of responsiveness is unsatisfactory, increase a strength of the pacing signal responsively to the detected at least one cardiac parameter.

In one embodiment of the valve prosthesis system,
the at least one cardiac parameter includes at least one timing feature,
the parameters of the pacing signal include at least one timing parameter, and
the non-implantable control circuitry is configured to set the at least one timing parameter of the pacing signal responsively to the at least one timing feature of the detected at least one cardiac parameter.

In the valve prosthesis system, the prosthetic aortic valve may include one or more elongate insulated electrical conductors, which directly couple the prosthetic-valve coil in the non-wireless electrical communication with the cathode and the anode.

In the valve prosthesis system, respective non-electrically-insulated end portions of the prosthetic-valve coil may define the cathode and the anode.

In the valve prosthesis system, the non-implantable control circuitry may be configured to set an amplitude of the pacing signal by modulating an amplitude of the energy wirelessly transferred from the energy-transmission coil to the prosthetic-valve coil.

In one embodiment of the valve prosthesis system, the pacing signal includes pulses, and the non-implantable control circuitry is configured to drive the cathode and the anode to (a) begin application of each pulse of the pacing signal by beginning wirelessly transferring energy from the energy-transmission coil to the prosthetic-valve coil, and (b) conclude the application of each pulse of the pacing signal by ceasing wirelessly transferring energy from the energy-transmission coil to the prosthetic-valve coil.

In the valve prosthesis system, the non-implantable unit may be an external unit, which is configured to be disposed outside a body of the patient.

In one embodiment of the valve prosthesis system, the non-implantable unit is a delivery system, which further includes a delivery tube, and one or more wires, which pass along the delivery tube, the energy-transmission coil is a delivery-system coil, the non-implantable control circuitry is delivery-system control circuitry, which is in electrical communication with the delivery-system coil via the one or more wires, and the delivery-system coil is coupled to the delivery tube at a distal site of the delivery tube. In such an embodiment, the delivery-system control circuitry may be configured to drive the cathode and the anode to apply rapid ventricular pacing, by wirelessly transferring the energy from the energy-transmission coil to the prosthetic-valve coil by inductive coupling.

In one embodiment of the valve prosthesis system, the non-implantable control circuitry is configured to wirelessly transfer the energy by generating a plurality of AC pulses, each including a train of AC bursts, and the prosthetic aortic valve includes a passive diode, which is coupled in electrical communication with the prosthetic-valve coil, and is configured to rectify current in the prosthetic-valve coil. In such an embodiment, the non-implantable control circuitry may be configured to generate the train of AC bursts at a frequency of between 3 kHz and 130 kHz, such as between 3 kHz and 100 kHz, or between 100 kHz and 130 kHz. In such an embodiment, the non-implantable control circuitry may be configured to include 20-100 AC bursts in each of the AC pulses.

The present invention will be more fully understood from the following detailed description of embodiments thereof, taken together with the drawings, in which:

### BRIEF DESCRIPTION OF THE DRAWINGS

Figs. 1A and 1B are schematic illustrations of a prosthetic aortic valve;
Fig. 2 is a schematic illustration of components of the prosthetic aortic valve of Figs. 1A-B before complete assembly;
Fig. 3A is a schematic illustration of another prosthetic aortic valve, in accordance with an application of the present invention;
Fig. 3B is a schematic illustration of passive electrical components of the prosthetic aortic valve of Fig. 3A and cardiac tissue, in accordance with an application of the present invention;
Figs. 4A-C are schematic illustrations of a valve prosthesis system, in accordance with respective applications of the present invention, and a method of using the system; and
Fig. 5 is a schematic illustration of an electronic implant.

### DETAILED DESCRIPTION OF APPLICATIONS

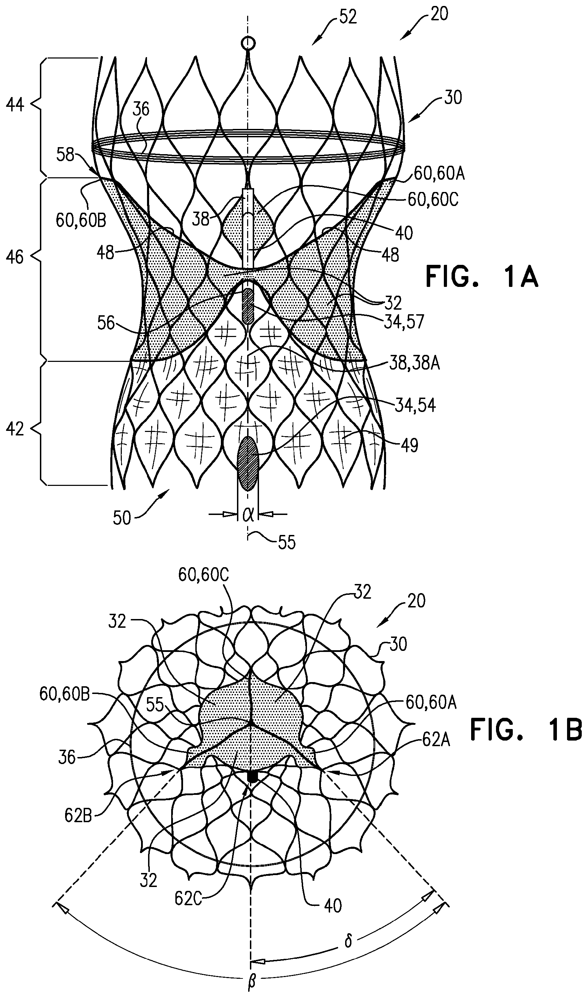

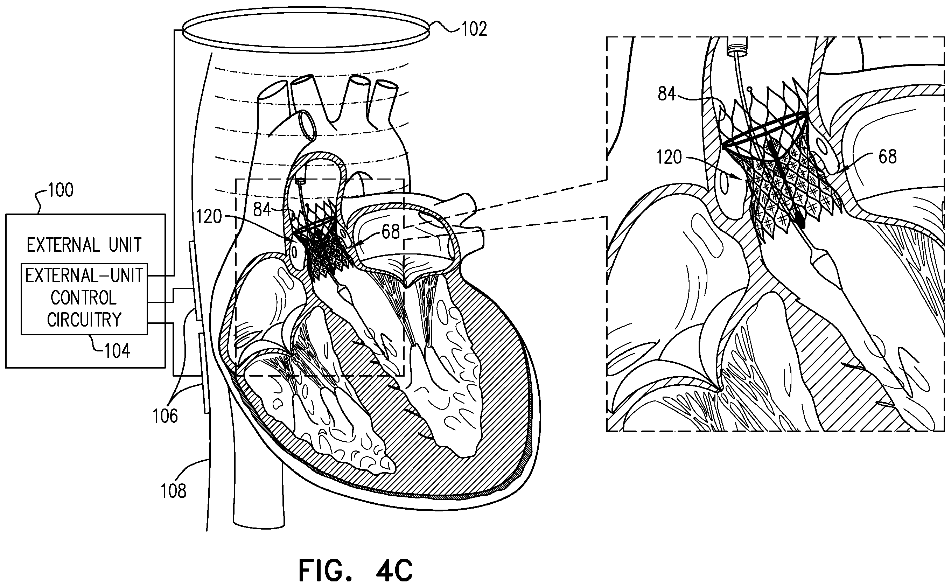

Figs. 1A and 1B are schematic illustrations of a prosthetic aortic valve 20. Prosthetic aortic valve 20 is shown in Figs. 1A-B in an expanded configuration, which is similar to the expanded fully-deployed configuration described hereinbelow with reference to Fig. 4C, except that in Figs. 1A-B expansion of prosthetic aortic valve 20 is not limited by anatomy of a patient. Fig. 1B is a view of prosthetic aortic valve 20 from a downstream outflow end 52, as described hereinbelow.

Prosthetic aortic valve 20 comprises:
- a frame 30;
- a plurality of prosthetic leaflets 32 coupled to frame 30;
- one or more electrodes 34 coupled to frame 30; and
- a prosthetic-valve coil 36 coupled to frame 30 and in non-wireless electrical communication with the one or more electrodes 34, optionally by one or more elongate insulated electrical conductors 38, e.g., wires.

Frame 30 typically comprises a stent or other structure, which is typically self-expanding, and may be formed by laser cutting or etching a metal alloy tube comprising, for example, stainless steel or a shape memory material such as Nitinol. For some applications, one or more of electrodes 34 are coupled to frame 30 using techniques described in US Patent 9,526,637 to Dagan et al. and/or US 2016/0278951 to Dagan et al. For some applications, prosthetic-valve coil 36 comprises gold wire, in order to provide low resistance.

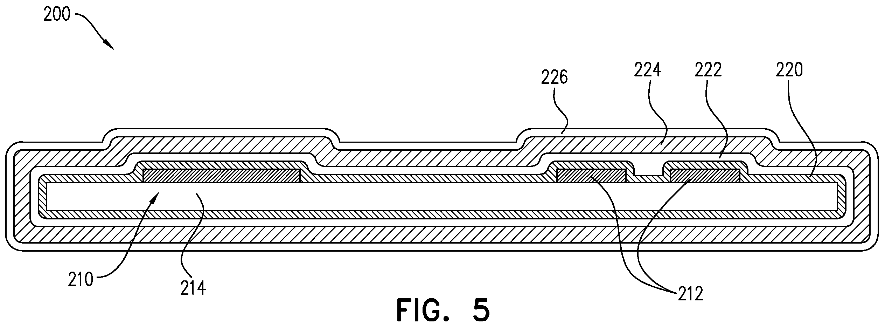

For some applications, prosthetic aortic valve 20 further comprises prosthetic-aortic-valve control circuitry 40, which is coupled to frame 30 and which is in non-wireless electrical communication with the one or more electrodes 34. In these applications, prosthetic-valve coil 36 is in non-wireless electrical communication with prosthetic-aortic-valve control circuitry 40, such that prosthetic-valve coil 36 is in non-wireless electrical communication with the one or more electrodes 34 via prosthetic-aortic-valve control circuitry 40. One or more of the one or more electrodes 34 may be directly attached in non-wireless electrical communication to prosthetic-aortic-valve control circuitry 40, and/or may be attached in non-wireless electrical communication to prosthetic-aortic-valve control circuitry 40 by the one or more elongate insulated electrical conductors 38. Typically, prosthetic-aortic-valve control circuitry 40 is flexible, and has a thin, linear packaging, and may implement techniques described hereinbelow with reference to Fig. 5. The thinness of control circuitry 40 allows it to be compressed in a delivery tube during deployment of prosthetic aortic valve 20, without the need to increase the diameter of the delivery tube. In addition, the flexibility of control circuitry 40 prevents damage to the control circuitry when it is crimped when compressed into the delivery tube.

For some applications, frame 30 is shaped so as to define an upstream inflow portion 42, a downstream outflow portion 44, and a constriction portion 46, which is axially between upstream inflow portion 42 and downstream outflow portion 44. Prosthetic leaflets 32 are coupled to constriction portion 46 such that free edges 48 of prosthetic leaflets 32 face toward downstream outflow portion 44 when prosthetic aortic valve 20 is in the expanded fully-deployed configuration described hereinbelow with reference to Fig. 4C. Prosthetic leaflets 32 are not coupled to downstream outflow portion 44; therefore, a ring-shaped longitudinal border 58 between downstream outflow portion 44 and constriction portion 46 is defined by a downstream-most point of frame 30 to which prosthetic leaflets 32 are coupled (for example, prosthetic leaflets 32 may be coupled to the downstream-most point of frame 30 at commissures 60, described immediately hereinbelow). (Ring-shaped longitudinal border 58 is at the same longitudinal location around frame 30.) Typically, prosthetic aortic valve 20 further comprises a skirt 49 coupled to upstream inflow portion 42 of frame 30, and prosthetic leaflets 32 are attached along their bases to skirt 49, for example, using sutures or a suitable biocompatible adhesive. Adjoining pairs of leaflets are attached to one another at their lateral ends to form commissures 60, with free edges 48 of the prosthetic leaflets forming coaptation edges that meet one another. Skirt 49 and prosthetic leaflets 32 typically comprise a sheet of animal pericardial tissue, such as porcine pericardial tissue, or synthetic or polymeric material.

For some applications, prosthetic-valve coil 36 is disposed no more than 1 mm upstream of ring-shaped longitudinal border 58 between downstream outflow portion 44 and constriction portion 46, typically axially along downstream outflow portion 44. Such placement allows prosthetic aortic valve 20 to be crimped (compressed) into a delivery tube during deployment of prosthetic aortic valve 20, without requiring a larger-diameter delivery tube to accommodate prosthetic-valve coil 36. This is possible because downstream outflow portion 44 does not include material of prosthetic leaflets 32, and thus can accommodate prosthetic-valve coil 36 without causing downstream outflow portion 44 to have a greater compressed diameter than the other axial portions of prosthetic aortic valve 20. Typically, prosthetic-valve coil 36 is not disposed axially along constriction portion 46 and is not disposed axially along upstream inflow portion 42. In addition, placement of prosthetic-valve coil 36 axially along downstream outflow portion 44 improves transmission efficiency because downstream outflow portion 44 typically has a greater diameter than each of constriction portion 46 and upstream inflow portion 42. In addition, constriction portion 46 typically has a lesser diameter than each of upstream inflow portion 42 and downstream outflow portion 44.

Typically, at least one of the one or more electrodes 34 is coupled to upstream inflow portion 42 of frame 30, such as exactly one of the one or more electrodes 34. For some applications, the one or more electrodes 34 comprise a cathode 54 that is coupled to upstream inflow portion 42 of frame 30, and prosthetic-aortic-valve control circuitry 40 is configured to drive cathode 54 to apply a cathodic current. For some applications, cathode 54 has a lateral dimension α (alpha), measured in degrees around frame 30 with respect to a central longitudinal axis 55 of frame 30, of between 10 and 40 degrees, e.g., between 20 and 40 degrees, such as 30 degrees, in order to accommodate rotational misplacement of frame 30 with respect to the bundle of His. Typically, prosthetic aortic valve 20 is deployed using imaging, such as fluoroscopy, and is rotated if necessary during the deployment such that cathode 54 is disposed against tissue of the annulus that is near the bundle of His. For some applications, prosthetic aortic valve 20 comprises a plurality of cathodes 54 (e.g., two or three, or more), which are disposed at a respective plurality of angular locations around frame 30 (e.g., 10-15 degrees apart). After implantation of prosthetic aortic valve 20, the cathode 54 that is has the most accurate angular location is activated to apply a pacing signal and/or sense, either by prosthetic-aortic-valve control circuitry 40 or external control circuitry, such as external-unit control circuitry 104, described hereinbelow with reference to Fig. 4C. Alternatively or additionally, for some applications, cathode 54 has an axial length of at least 10 mm, in order to accommodate axial misplacement of frame 30 with respect to the annulus of the natural aortic valve, and thus with respect to the bundle of His. As used in the present application, including in the claims, an "axial length" is a length of a structure measured along central longitudinal axis 55.

For some applications, cathode 54 has a thickness of between 75 and 125 microns, e.g., about 100 microns, and/or a surface area of at least 2.5 mm2, in order to provide adequate stimulation. For some applications, cathode 54 comprises titanium nitride (TiN). For some applications, skirt 49 is coupled to an external surface of upstream inflow portion 42 of frame 30, and cathode 54 is disposed on an external surface of skirt 49. As used in the present application, including in the claims, the "central longitudinal axis" 55 of frame 30 is the set of all centroids of transverse cross-sectional sections of frame 30 along frame 30. Thus the cross-sectional sections are locally perpendicular to the central longitudinal axis, which runs along frame 30. (For applications in which frame 30 is circular in cross-section, the centroids correspond with the centers of the circular cross-sectional sections.)

For some applications, when prosthetic aortic valve 20 is in the expanded fully-deployed configuration described hereinbelow with reference to Fig. 4C:
- frame 30 has an inflow end 50 at upstream inflow portion 42 and downstream outflow end 52 at downstream outflow portion 44, and an axial length, measured between inflow end 50 and downstream outflow end 52, and
- at least one of (e.g., exactly one of, e.g., cathode 54) the one or more electrodes 34 is coupled to upstream inflow portion 42 within a distance from inflow end 50, the distance equal to 10% of the axial length of frame 30 (the distance is measured (a) along central longitudinal axis 55 of frame 30 when in the expanded fully-deployed configuration, and (b) between inflow end 50 and an upstream-most point of the at least one electrode).

Typically, prosthetic-aortic-valve control circuitry 40 is coupled to frame 30 such that upstream-most point 56 of prosthetic-aortic-valve control circuitry 40 is disposed axially along constriction portion 46 and/or downstream outflow portion 44 of frame 30.

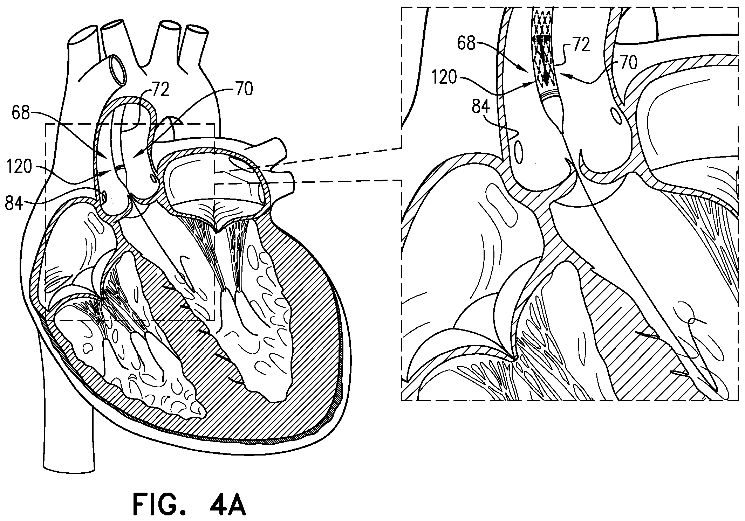

Typically, prosthetic-aortic-valve control circuitry 40 is coupled to frame 30 inside frame 30, which may prevent friction between prosthetic-aortic-valve control circuitry 40 and delivery tube 72 during deployment of prosthetic aortic valve 20, described hereinbelow with reference to Figs. 4A-C. It is noted that for applications in which upstream-most point 56 is disposed no more upstream than 1 mm upstream of ring-shaped longitudinal border 58, such as described above, there is generally enough space inside frame 30 to accommodate prosthetic-aortic-valve control circuitry 40.

For some applications, prosthetic leaflets 32 are coupled to frame 30 at at least first and second commissures 60A and 60B of prosthetic aortic valve 20 that are located at respective first and second angular locations 62A and 62B around frame 30. The first and second angular locations 62A and 62B are separated by a first angular offset β (beta) around frame 30 when prosthetic aortic valve 20 is in the expanded fully-deployed configuration described hereinbelow with reference to Fig. 4C. Prosthetic-aortic-valve control circuitry 40 is coupled to frame 30 at a third angular location 62C around frame 30 that is separated from first angular location 62A by a second angular offset δ (delta) that equals between 40% and 60% (e.g., 50%) of the first angular offset β (beta) when prosthetic aortic valve 20 is in the expanded fully-deployed configuration described hereinbelow with reference to Fig. 4C. At the third angular location 62C around frame 30, the frame is more flexible than at the more rigid commissures. As used in the present application, including in the claims, an "angular location" is a location on frame 30 at a particular location around central longitudinal axis 55, i.e., at a particular "o'clock" with respect to central longitudinal axis 55. (It is noted that a third commissure 60C is shown in Fig. 1A on the far side of the frame, i.e., 180 degrees from circuitry 40.)

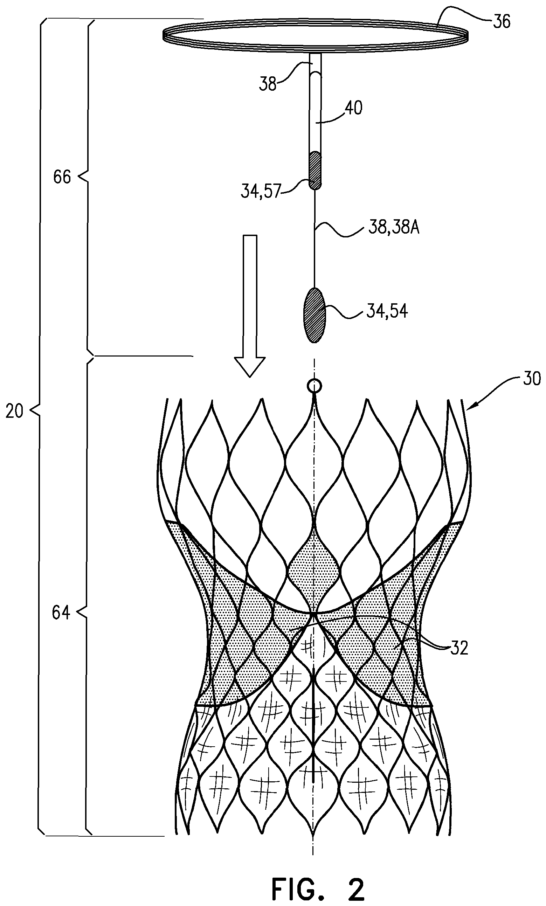

Reference is now made to Fig. 2, which is a schematic illustration of components of prosthetic aortic valve 20 before complete assembly, in accordance with an application of the present invention. The components comprise a valve component 64 and an electronics component 66. Valve component 64 typically consists of a heart valve prosthesis known in the art, which comprises at least frame 30 and prosthetic leaflets 32. For example, the known heart valve prosthesis may comprise a CoreValveTM EvolutTM R prothesis (Medtronic, Inc., Minneapolis, MN, USA), a CoreValveTM EvolutTM PRO prosthesis (Medtronic, Inc.), a LOTUS EdgeTM Aortic Valve (Boston Scientific Corporation, Marlborough, MA, USA), or an ACURATE neoTM Aortic Valve (Boston Scientific Corporation). Electronics component 66 comprises at least the one or more electrodes 34 and prosthetic-valve coil 36, and optionally prosthetic-aortic-valve control circuitry 40.

During assembly of prosthetic aortic valve 20, electronics component 66 is inserted into valve component 64. For some applications, a first portion of electronics component 66, such as prosthetic-valve coil 36, prosthetic-aortic-valve control circuitry 40, and one of the one or more electrodes 34, is coupled to an inner surface of frame 30, and a second portion of electronics component 66, such as cathode 54, is coupled to an external surface of frame 30. For example, one 38A of one or more elongate insulated electrical conductors 38 may electrically couple cathode 54 to prosthetic-aortic-valve control circuitry 40, and the conductor 38A may pass from inside to outside frame 30, typically through skirt 49. (Coupling one of the one or more electrodes 34 to the inner surface of frame 30 may expose the electrode to blood of the subject upon implantation of the assembled prosthetic aortic valve 20. Coupling cathode 54 to the external surface of frame 30 may dispose the cathode against tissue, such as tissue of the annulus that is near the bundle of His, upon implantation of the assembled prosthetic aortic valve 20, such as described herein.) Optionally, the components of electronics component 66 may be stitched to frame 30 and/or skirt 49.

For some applications, whether prosthetic-valve coil 36 is coupled to an inner or an external surface of frame 30, prosthetic-valve coil 36 is electrically isolated from frame 30, such as by isolation material (e.g., a sheet of material or a coating) disposed between prosthetic-valve coil 36 and frame 30. For example, the isolation material may comprise a non-conductive polymer.

The above-mentioned assembly of prosthetic aortic valve 20 is typically performed in a manufacturing facility, and thereafter the assembled prosthetic aortic valve 20 is packaged and shipped to a healthcare facility for implantation. The method of assembling prosthetic aortic valve 20 is thus non-surgical.

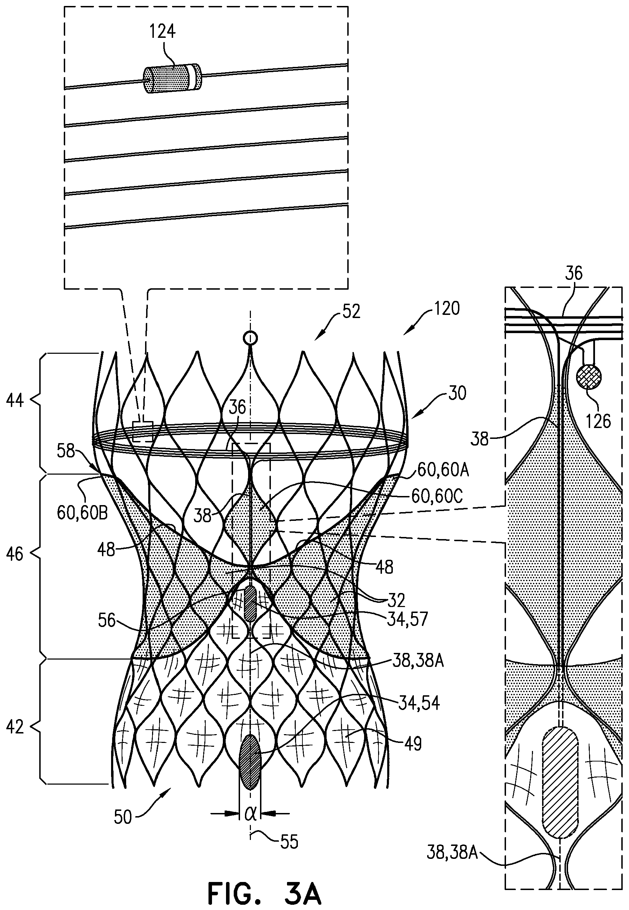

Fig. 3A is a schematic illustration of a prosthetic aortic valve 120, in accordance with an application of the present invention. Prosthetic aortic valve 120 is shown in Fig. 3A in an expanded configuration, which is similar to the expanded fully-deployed configuration of prosthetic aortic valve 20 described hereinbelow with reference to Fig. 4C, except that in Fig. 3A expansion of prosthetic aortic valve 120 is not limited by anatomy of a patient. Other than as described hereinbelow, prosthetic aortic valve 120 is identical to prosthetic aortic valve 20 described herein with reference to Figs. 1A-B and 2, and like reference numerals refer to like parts. Prosthetic aortic valve 120 may be assembled as described hereinabove with reference to Fig. 2 for prosthetic aortic valve 20, *mutatis mutandis.*

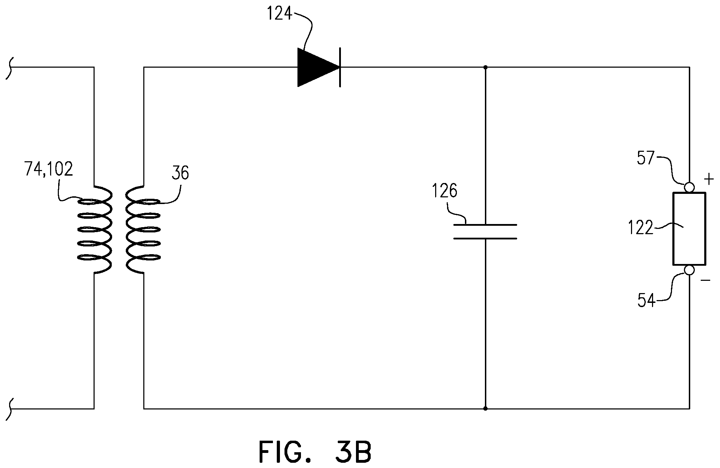

Reference is also made to Fig. 3B, which is a schematic illustration of passive electrical components of prosthetic aortic valve 120 and tissue 122, in accordance with an application of the present invention. Tissue 122 includes cardiac tissue and blood. Cathode 54 is configured to be in contact with the cardiac tissue, and anode 57 is configured to be in contact with the blood. As is known in the art, cardiac tissue acts as a resistor.

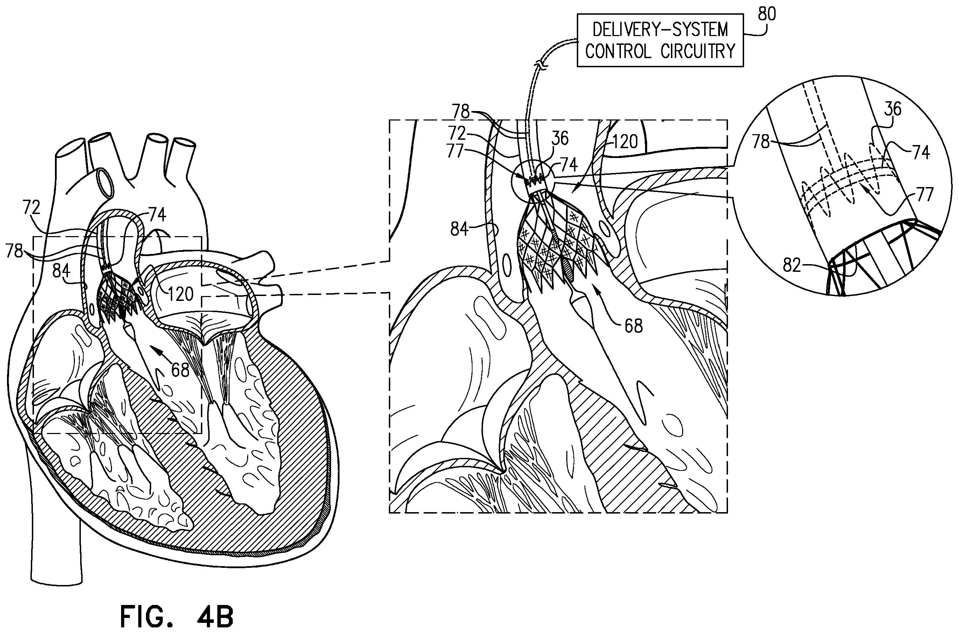

For some applications, prosthetic aortic valve 120 comprises a passive diode 124 (shown highly schematically in the upper exploded view in Fig. 3A, as well as in Fig. 3B), which is coupled in electrical communication with prosthetic-valve coil 36 and rectifies current in the prosthetic-valve coil. For example, diode 124 may be positioned at one end of the coil or adjacent to cathode 54 or anode 57, or (as shown in Fig. 3A) at some point along prosthetic-valve coil 36. Non-implantable control circuitry (such as delivery-system control circuitry 80 (Fig. 4B) or external-unit control circuitry 104 (Fig. 4C)) typically wirelessly transfers energy to prosthetic-valve coil 36 by generating a plurality of AC pulses, each AC pulse including a train of AC bursts. The train of AC bursts may be generated, for example, at a frequency of between 3 kHz and 130 kHz (e.g., between 3 kHz and 100 kHz, or between 100 kHz and 130 kHz), for improved efficiency. For some applications, there are 20-100 AC bursts in each of the AC pulses. Other frequencies and number of bursts are within the scope of the present invention.

For some applications, prosthetic aortic valve 120 comprises exactly one passive diode 124, which provides half-wave rectification of the AC pulses. For other applications, prosthetic aortic valve 120 comprises a plurality of passive diodes 124, which provide full-wave rectification of the AC pulses; for example, prosthetic aortic valve 120 may comprise four passive diodes 124 arranged in a bridge configuration, as is known in the electronics art.

For some applications, prosthetic aortic valve 120 comprises a capacitor 126 (shown highly schematically in the exploded view to the right in Fig. 3A, as well as in Fig. 3B), which is in electrical communication with cathode 54 and anode 57 (parallel to cardiac tissue 122 in the circuit made upon implantation of the electrodes). Capacitor 126 typically increases the efficiency of the circuit by delivering a larger proportion of the received energy into tissue 122. (As is known in the electronics art, a capacitor is a passive electrical component.)

Optionally, prosthetic aortic valve 120 comprises additional passive electrical components, such as one or more resistors.

As described hereinbelow with reference to Fig. 4B regarding prosthetic aortic valve 20, for some applications, delivery-system control circuitry 80 is configured to drive the one or more electrodes 34 to apply the rapid ventricular pacing; in this configuration, prosthetic-aortic-valve control circuitry 40, if even provided, is generally passive, i.e., delivery-system control circuitry 80 sets the parameters of the pacing signal. Prosthetic aortic valve 120, shown in Fig. 3A, is one implementation of this configuration; unlike the configuration of prosthetic aortic valve 20 illustrated in Figs. 1A-B and 2, prosthetic aortic valve 120 does not comprise prosthetic-aortic-valve control circuitry 40 or any other active electronic components.

A valve prosthesis system is provided that comprises (a) prosthetic aortic valve 120 and (b) a non-implantable unit, such as delivery system 70, described hereinbelow with reference to Figs. 4A-C, or external unit 100, described hereinbelow with reference to Fig. 4C. Non-implantable control circuitry (such as delivery-system control circuitry 80 or external-unit control circuitry 104 of external unit 100, as appropriate) is configured to drive cathode 54 and anode 57 to apply a pacing signal and to set parameters of the pacing signal (e.g., to be a standard, chronic pacing signal, or a rapid ventricular pacing signal), by wirelessly transferring energy from an energy-transmission coil (such as delivery-system coil 74 or external-unit coil 102, described hereinbelow with reference to Fig. 4C, as appropriate) to prosthetic-valve coil 36 by inductive coupling. The applied pacing is typically bipolar.

Optionally, the valve prosthesis system comprises two non-implantable units: (1) delivery system 70, described hereinbelow with reference to Figs. 4A-C, and (2) external unit 100, described hereinbelow with reference to Fig. 4C, which comprise respective control circuitry and energy-transmission coils. Delivery-system control circuitry 80 is configured to drive delivery-system coil 74 to drive cathode 54 and anode 57 to apply the pacing signal and to set the parameters of the pacing signal, by wirelessly transferring energy, by inductive coupling, to prosthetic-valve coil 36 when prosthetic aortic valve 120 is in the partially-deployed configuration, such as described hereinbelow with reference to Fig. 4B. External-unit control circuitry 104 is configured to drive external-unit coil 102, described hereinbelow with reference to Fig. 4C, to drive cathode 54 and anode 57 to apply the pacing signal and to set the parameters of the pacing signal, by wirelessly transferring energy, by inductive coupling, to prosthetic-valve coil 36 when prosthetic aortic valve 120 is in the expanded fully-deployed configuration, such as described hereinbelow with reference to Fig. 4C.

Typically, respective ends of prosthetic-valve coil 36 are in the non-wireless electrical communication with cathode 54 and anode 57.

For some applications, respective non-electrically-insulated end portions of prosthetic-valve coil 36 define cathode 54 and anode 57. In these applications, prosthetic aortic valve 120 typically does not comprise elongate insulated electrical conductors 38. Instead, respective insulated end portions of prosthetic-valve coil 36 bend away from prosthetic-valve coil 36 along the paths of elongate insulated electrical conductors 38 shown in Fig. 3A, such that the respective non-electrically-insulated end portions of prosthetic-valve coil 36 are located at the locations at which cathode 54 and anode 57 are shown in Fig. 3A, respectively.

As mentioned above, the non-implantable control circuitry is configured to drive cathode 54 and anode 57 to set parameters of the pacing signal. For example, the non-implantable control circuitry may be configured to set an amplitude of the pacing signal by modulating an amplitude of the energy wirelessly transferred from the energy-transmission coil to prosthetic-valve coil 36. Alternatively or additionally, for example, the non-implantable control circuitry may be configured to drive cathode 54 and anode 57 to (a) begin application of each pulse of the pacing signal by beginning wirelessly transferring energy from the energy-transmission coil to prosthetic-valve coil 36, and (b) conclude the application of each pulse of the pacing signal by ceasing wirelessly transferring energy from the energy-transmission coil to prosthetic-valve coil 36.

The inventor has determined that, in some configurations, it is difficult to assess suitable pacing parameters, e.g., due to patient size or patient body mass distribution, or for example due to technical issues such as variable electrical impedance between heart tissue and cathode 54 and anode 57, or the variable relative orientation of external-unit coil 102 and prosthetic-valve coil 36. For some applications, therefore, the non-implantable unit comprises an energy-transmission coil (e.g., external-unit coil 102, as shown in Fig. 4C), and at least two sensing skin ECG electrodes 106, placed on the patient's skin 108, e.g., on the chest as shown in Fig. 4C. The non-implantable control circuitry (e.g., external-unit control circuitry 104) drives cathode 54 and anode 57 to apply a pacing signal to the patient's heart, and to detect at least one cardiac parameter using sensing skin ECG electrodes 106. The non-implantable control circuitry, at least partially responsively to the detected cardiac parameter, sets parameters of the pacing signal, by wirelessly transferring energy from the energy-transmission coil to prosthetic-valve coil 36 by inductive coupling. Because prosthetic aortic valve 120 typically does not comprise any active electronic components, the wireless transfer of energy from the energy-transmission coil to prosthetic-valve coil 36 by inductive coupling itself inductively drives the pacing current through prosthetic-valve coil 36.

Alternatively, in arrangements not embodying the present invention, the non-implantable unit comprises another type of cardiac sensor, instead of sensing skin ECG electrodes 106. For example, the cardiac sensor may comprise a heart rate sensor, such as an optical heart rate sensor (e.g., which uses photoplethysmography), or an ECG sensor, such as an optical ECG sensor (e.g., a single channel ECG sensor, such as the Si1172 or Si1173 biometric modules, manufactured by Silicon Laboratories Inc., Austin, TX, USA).

The non-implantable control circuitry typically analyzes the detected cardiac parameter to assess a level of responsiveness of the heart to the pacing signal. Upon ascertaining that the level of responsiveness is unsatisfactory, the non-implantable control circuitry increases the strength of the pacing signal responsively to the detected cardiac parameter (e.g., by increasing the amplitude or the duration of the pacing signal). For example, the pulse width (typically 0.1 - 1 ms, e.g., 0.25 - 0.8 ms) of pulses of the pacing signal, or current amplitude in the energy-transmission coil may be iteratively increased, until a determination is made that the heart is suitably responding to the pacing pulses applied to the tissue. At this point, optionally, the strength of the pacing signal is further increased, e.g., by 50-150%, for example by 100%.

For some applications, the detected cardiac parameter is a timing feature of cardiac activity (e.g., heart rate, or the timing of a particular feature of the cardiac cycle). In this case, the parameters of the pacing signal may include a timing parameter of the pacing signal, and the non-implantable control circuitry sets the timing parameter of the pacing signal responsively to the timing feature of the detected cardiac parameter.

It is noted that, as appropriate for a given patient, pacing of the heart may be applied in a manner that is synchronized to the cardiac cycle of the patient (based on the signals received by sensing skin ECG electrodes 106), or the pacing may not be synchronized with the cardiac cycle of the patient.

Sensing skin ECG electrodes 106 are typically suction ECG electrodes or configured to be electrically coupled to the skin by an adhesive. In general, conventional ECG electrodes are suitable to be used for sensing skin ECG electrodes 106. It is noted that although conventional ECG electrodes may be used, complete ECG analysis as is known in the field of electrocardiography typically is not performed in order to implement the functions of the non-implantable control circuitry described hereinabove.

Reference is made to Figs. 1A-B, 2, and 3A, and is additionally made to Figs. 4A-C, which are schematic illustrations of a valve prosthesis system 68 and a method of using the system. Although the techniques described with reference to Figs. 4A-C are generally described regarding prosthetic aortic valve 120, the techniques are equally applicable to prosthetic aortic valve 20, *mutatis mutandis.* The rotational orientation of the prosthetic aortic valve is shown schematically in Figs. 4A-C, in order to illustrate the components of the prosthetic aortic valve; as described below, in actual use, the prosthetic aortic valve is typically rotationally oriented such that cathode 54 is positioned adjacent to cardiac tissue near the bundle of His.

Valve prosthesis system 68 comprises prosthetic aortic valve 20 or prosthetic aortic valve 120 and a delivery system 70.

Delivery system 70 comprises:
- a delivery tube 72;
- a delivery-system coil 74, which is coupled to delivery tube 72 at a distal site 76 of delivery tube 72; for example, a distal-most portion 77 of delivery-system coil 74 may be disposed within 10 mm of a distal end 82 of delivery tube 72;
- one or more wires 78, which pass along delivery tube 72, e.g., attached to an outer or inner surface of delivery tube 72, or embedded in the wall of delivery tube 72; and
- delivery-system control circuitry 80, which is in electrical communication with delivery-system coil 74 via the one or more wires 78.

Delivery-system control circuitry 80 is configured to drive delivery-system coil 74 to wirelessly transfer energy, by inductive coupling, to prosthetic-valve coil 36 at least when prosthetic aortic valve 120 is in the partially-deployed configuration described hereinbelow with reference to Fig. 4B.

As shown in Fig. 4A, prosthetic aortic valve 120 is removably disposable in delivery tube 72 in a compressed delivery configuration. During an implantation procedure, delivery tube 72 is advanced through vasculature of a patient, until distal end 82 of delivery tube 72 is disposed in an ascending aorta 84 of the patient, while prosthetic aortic valve 120 is removably disposed in delivery tube 72 in the compressed delivery configuration.

As shown in Fig. 4B, prosthetic aortic valve 120 is also configured to assume a partially-expanded partially-deployed configuration upon being partially released from distal end 82 of delivery tube 72 such that (a) at least one of the one or more electrodes 34 is positioned outside delivery tube 72, such as cathode 54, in the vicinity of (e.g., touching) target tissue, such as the natural aortic valve annulus, and (b) prosthetic-valve coil 36 is compressed within delivery tube 72. Typically, delivery-system coil 74 surrounds compressed prosthetic-valve coil 36, which provides high transmission efficiency even though prosthetic-valve coil 36 is still compressed. After prosthetic aortic valve 120 has assumed the partially-expanded partially-deployed configuration, delivery-system control circuitry 80 is activated to drive delivery-system coil 74 to wirelessly transfer energy, by inductive coupling, to prosthetic-valve coil 36. By contrast, transmission of power from an external coil to compressed prosthetic-valve coil 36 would be quite inefficient because of the greater distance between the transmitting and receiving coils and the compression of prosthetic-valve coil 36.

For some applications in which valve prosthesis system 68 comprises prosthetic aortic valve 20, described hereinabove with reference to Figs. 1A-B and 2, prosthetic-aortic-valve control circuitry 40 is configured to drive the one or more electrodes 34 to apply rapid ventricular pacing. Such pacing may temporarily reduce left ventricular output, in order to enable more accurate placement of prosthetic aortic valve 20. Alternatively, such as described hereinabove with reference to Fig. 3A, delivery-system control circuitry 80 is configured to drive the one or more electrodes 34 to apply the rapid ventricular pacing; in this configuration, prosthetic-aortic-valve control circuitry 40, if even provided (as in prosthetic aortic valve 20), is generally passive, or prosthetic-aortic-valve control circuitry 40 is not provided (as in prosthetic aortic valve 120), i.e., delivery-system control circuitry 80 sets the parameters of the pacing signal. Alternatively, prosthetic aortic valve 20 or 120 is not used for applying rapid ventricular pacing, and may instead be used for applying pacing post-implantation, such as described below, and/or for post-implantation sensing, such as described below.

As described hereinabove with reference to Figs. 1A-B, for some applications, the one or more electrodes 34 comprise cathode 54 that is coupled to upstream inflow portion 42 of frame 30. When prosthetic aortic valve 120 is in the partially-expanded partially-deployed configuration shown in Fig. 4B, cathode 54 is positioned adjacent to cardiac tissue near the bundle of His, in order to pace the heart by stimulating the cardiac tissue with cathodic current. For some applications, the one or more electrodes further comprise an anode 57, which may be used for bipolar sensing and/or pacing, as known in the art. Typically, cathode 54 and anode 57 are disposed on frame 30 such that there is at least 15 mm between the cathode and the anode, when prosthetic aortic valve 120 is in the expanded fully-deployed configuration described hereinbelow with reference to Fig. 4C (the 15 mm is measured along central longitudinal axis 55 of frame 30 when in the expanded fully-deployed configuration).

As shown in Fig. 4C, prosthetic aortic valve 120 is also configured to assume an expanded fully-deployed configuration upon being fully released from distal end 82 of delivery tube 72. For some applications, delivery-system control circuitry 80 is configured to cease driving delivery-system coil 74 to wirelessly transfer the energy when prosthetic aortic valve 120 assumes the expanded fully-deployed configuration upon being fully released from distal end 82 of delivery tube 72.

For some applications, as shown in Fig. 4C, valve prosthesis system 68 further comprises an external unit 100, which comprises (a) an external-unit coil 102, and (b) external-unit control circuitry 104, which is configured to drive external-unit coil 102 to wirelessly transfer energy, by inductive coupling, to prosthetic-valve coil 36 when prosthetic aortic valve 120 is in the expanded fully-deployed configuration. In these applications, after prosthetic aortic valve 120 is fully released from distal end 82 of delivery tube 72, external-unit control circuitry 104 is activated to drive external-unit coil 102 to wirelessly transfer energy, by inductive coupling, to prosthetic-valve coil 36 when prosthetic aortic valve 120 is in the expanded fully-deployed configuration.

For some applications, external-unit coil 102 is incorporated into a collar configured to be worn around the patient's neck or placed on the patient's chest, such as described in PCT Publication WO 2016/157183 to Dagan et al., and/or incorporated into a band configured to be worn around the patient's chest or a necklace configured to be worn around the patient's neck. This positioning of external-unit coil 102 provides high transmission efficiency, because the respective axes of external-unit coil 102 and prosthetic-valve coil 36 are generally aligned.

Alternatively or additionally, for some applications, external unit 100 is incorporated into a belt or strap configured to be worn around the patient's chest.

For some applications in which valve prosthesis system 68 comprises prosthetic aortic valve 20, described hereinabove with reference to Figs. 1A-B and 2, prosthetic-aortic-valve control circuitry 40 is configured to use the received energy to drive the one or more electrodes 34 to perform pacing post-implantation, e.g., for several months. Such pacing may employ any standard pacing protocol. For some applications, the pacing is VVI pacing, which is only applied when a QRS complex is not sensed in the ventricle. Alternatively, for some applications in which valve prosthesis system 68 comprises prosthetic aortic valve 120, described hereinabove with reference to Fig. 3A, external-unit control circuitry 104 is configured to drive the one or more electrodes 34 to apply the pacing signal; in this configuration, prosthetic-aortic-valve control circuitry 40 is not provided (or if provided, is generally passive), i.e., external-unit control circuitry 104 sets the parameters of the pacing signal.

Alternatively, for some applications in which valve prosthesis system 68 comprises prosthetic aortic valve 20, described hereinabove with reference to Figs. 1A-B and 2, prosthetic-aortic-valve control circuitry 40 is configured to (a) use the one or more electrodes 34 to sense a cardiac signal, and (b) drive prosthetic-valve coil 36 to transmit a wireless signal indicative of the sensed cardiac signal. For some applications, the cardiac sensing is performed using techniques described in US Patent 9,005,106 to Gross et al.. In these applications, the one or more electrodes 34 are typically not used to apply pacing, any thus need not be configured as a cathode and an anode. Such sensing may enable early discharge of the patient from the hospital after implantation of prosthetic aortic valve 20, before the possible development of left bundle branch block (LBBB). If LBBB develops, as it does in approximately 20-30% of patients, the LBBB is detected by the sensing, an alert is generated, and the LBBB may be treated as appropriate.

Reference is now made to Fig. 5, which is a schematic illustration of an electronic implant 200. Prosthetic-aortic-valve control circuitry 40, described hereinabove with reference to Figs. 1A-2, may implement features of electronic implant 200.

Electronic implant 200 comprises circuitry 210, which comprises electronic components 212, typically mounted on a long and flexible printed circuit board (PCB) 214. Electronic implant 200 further comprises a multi-layer protective coating, which comprises the following layers in the following order:
- a first inner aluminum oxide (AlOx) film layer 220 deposited on circuitry 210, e.g., using atomic layer deposition (ALD);
- a second parylene layer 222 deposited (typically, vapor-deposited in a vacuum) on first inner AlOx film layer 220; second parylene layer 222 provides chemical protection for circuitry 210;
- optionally, a third layer 224 disposed (typically cast onto) on second parylene layer 222, the third layer, for example, comprising a polymer, such as a polymer selected from the group consisting of: silicone and PTFE; third layer 224 typically has a thickness of between 100 and 200 microns, and is configured to provide mechanical protection for circuitry 210; and
- optionally, a fourth outer parylene layer 226 deposited (typically, vapor-deposited in a vacuum) on third layer 224; fourth outer parylene layer 226 provides chemical protection for circuitry 210 and third layer 224.

Electronic implant 200 and the layers are drawn highly schematically in Fig. 5, and are not drawn to scale; in particular, the layers are actually much thinner than shown, and the relative thicknesses are different from those shown.

Typically, circuitry 210 is not encased in a case, but is only coated with layers, as described above. A "case" is an enclosure, typically comprising glass and/or metal, that has a structure before circuitry is disposed therein; by contrast, a coating takes the shape of the circuitry to which the coating is applied. By contrast, encasement in a case is standard in the field of implantable circuitry. The lack of such a case allows electronic implant 200 to be thin and flexible, with the tradeoff of shorter lifespan. For prosthetic-aortic-valve control circuitry 40, the shorter lifespan is generally not an issue, because prosthetic-aortic-valve control circuitry 40 is typically only used for several months.

For applications in which prosthetic-aortic-valve control circuitry 40 implements features of electronic implant 200, the one or more electrodes 34 are masked during application of the coatings. Thus, prosthetic-aortic-valve control circuitry 40, the one or more elongate insulated electrical conductors 38 (e.g., wires), and prosthetic-valve coil 36 are all coated in the same coating procedure.

The techniques described herein for prosthetic aortic valve 20 may be alternatively used, *mutatis mutandis,* for non-aortic prosthetic valves, such as prosthetic mitral or tricuspid valves.

Techniques and apparatus described in one or more of the following patents and/or applications, which are assigned to the assignee of the present application, may be combined with techniques and apparatus described herein:
- US Patent 10,543,083 to Gross
- European Patent Application Publication EP 3508113 A1 to Gross
- US Patent 10,835,750 to Gross
- US Patent Application Publication 2020/0261224 to Gross
- an International Patent Application to Gross, filed on even date herewith, entitled, "Prosthetic Aortic Valve Pacing Systems" and published as WO 2021/140507

It will be appreciated by persons skilled in the art that the present invention is not limited to what has been particularly shown and described hereinabove. Rather, the scope of the present invention is defined by the appended claims, and may include both combinations and subcombinations of the various features described hereinabove, as well as variations and modifications thereof that are not in the prior art, which would occur to persons skilled in the art upon reading the foregoing description.

## Claims

1. A valve prosthesis system comprising:
(i) a prosthetic aortic valve (120), which is configured to be implanted in a native aortic valve of a patient, and which comprises:
(a) a plurality of prosthetic leaflets (32);
(b) a frame (30);
(c) a cathode (42) and an anode (57), which are mechanically coupled to the frame (30); and
(d) a prosthetic-valve coil (36), which is in non-wireless electrical communication with the cathode (42) and the anode (57), wherein the prosthetic aortic valve (120) does not comprise any active electronic components; and
(ii) a non-implantable unit, which comprises:
(a) an energy-transmission coil;
(b) at least two sensing skin ECG electrodes (106); and
(c) non-implantable control circuitry, which is configured to:
drive the cathode (42) and the anode (57) to apply a pacing signal to a heart of the patient,
detect at least one cardiac parameter using the at least two sensing skin ECG electrodes (106), and
at least partially responsively to the detected at least one cardiac parameter, set parameters of the pacing signal, by wirelessly transferring energy from the energy-transmission coil to the prosthetic-valve coil (36) by inductive coupling.

2. The valve prosthesis system according to claim 1, wherein the non-implantable control circuitry is configured to:
analyze the detected at least one cardiac parameter to assess a level of responsiveness of the heart to the pacing signal, and
upon ascertaining that the level of responsiveness is unsatisfactory, increase a strength of the pacing signal responsively to the detected at least one cardiac parameter.

3. The valve prosthesis system according to claim 1,
wherein the at least one cardiac parameter includes at least one timing feature,
wherein the parameters of the pacing signal include at least one timing parameter, and
wherein the non-implantable control circuitry is configured to set the at least one timing parameter of the pacing signal responsively to the at least one timing feature of the detected at least one cardiac parameter.

4. The valve prosthesis system according to claim 1, wherein the prosthetic aortic valve (120) comprises one or more elongate insulated electrical conductors (38), which directly couple the prosthetic-valve coil (36) in the non-wireless electrical communication with the cathode (42) and the anode (57).

5. The valve prosthesis system according to claim 1, wherein respective non-electrically-insulated end portions of the prosthetic-valve coil (36) define the cathode (42) and the anode (57).

6. The valve prosthesis system according to claim 1, wherein the non-implantable control circuitry is configured to set an amplitude of the pacing signal by modulating an amplitude of the energy wirelessly transferred from the energy-transmission coil to the prosthetic-valve coil (36).

7. The valve prosthesis system according to claim 1, wherein the pacing signal includes pulses, and wherein the non-implantable control circuitry is configured to drive the cathode (42) and the anode (57) to (a) begin application of each pulse of the pacing signal by beginning wirelessly transferring energy from the energy-transmission coil to the prosthetic-valve coil (36), and (b) conclude the application of each pulse of the pacing signal by ceasing wirelessly transferring energy from the energy-transmission coil to the prosthetic-valve coil (36).

8. The valve prosthesis system according to any one of claims 1-7, wherein the non-implantable unit is an external unit (100), which is configured to be disposed outside a body of the patient.

9. The valve prosthesis system according to any one of claims 1-7,
wherein the non-implantable unit is a delivery system (70), which further comprises a delivery tube (72), and one or more wires (78), which pass along the delivery tube (72),
wherein the energy-transmission coil is a delivery-system coil (74),
wherein the non-implantable control circuitry is delivery-system control circuitry (80), which is in electrical communication with the delivery-system coil (74) via the one or more wires (78), and
wherein the delivery-system coil (74) is coupled to the delivery tube (72) at a distal site (76) of the delivery tube (72).

10. The valve prosthesis system according to claim 9, wherein the delivery-system control circuitry (80) is configured to drive the cathode (42) and the anode (57) to apply rapid ventricular pacing, by wirelessly transferring the energy from the energy-transmission coil to the prosthetic-valve coil (36) by inductive coupling.

11. The valve prosthesis system according to any one of claims 1-7,
wherein the non-implantable control circuitry is configured to wirelessly transfer the energy by generating a plurality of AC pulses, each including a train of AC bursts, and
wherein the prosthetic aortic valve (120) comprises a passive diode (124), which is coupled in electrical communication with the prosthetic-valve coil (36), and is configured to rectify current in the prosthetic-valve coil (36).

12. The valve prosthesis system according to claim 11, wherein the non-implantable control circuitry is configured to generate the train of AC bursts at a frequency of between 3 kHz and 130 kHz.

13. The valve prosthesis system according to claim 11, wherein the non-implantable control circuitry is configured to include 20-100 AC bursts in each of the AC pulses.

14. The valve prosthesis system according to claim 11, wherein the prosthetic aortic valve (120) comprises a capacitor (126), which is in electrical communication with the cathode (42) and the anode (57).

## Patentansprüche

1. Klappenprothesensystem, umfassend:
(i) eine prothetische Aortenklappe (120), die konfiguriert ist, um in eine native Aortenklappe eines Patienten implantiert zu werden, und die Folgendes umfasst:
(a) eine Vielzahl von prothetischen Segeln (32);
(b) einen Rahmen (30);
(c) eine Kathode (42) und eine Anode (57), die mechanisch an den Rahmen (30) gekoppelt sind; und
(d) eine prothetische Klappenspule (36), die in nicht drahtloser elektrischer Kommunikation mit der Kathode (42) und der Anode (57) ist, wobei die prothetische Aortenklappe (120) keine aktiven elektronischen Komponenten umfasst; und
(ii) eine nicht implantierbare Einheit, die Folgendes umfasst:
(a) eine Energieübermittlungsspule;
(b) zumindest zwei Erfassungs-Haut-EKG-Elektroden (106); und
(c) nicht implantierbare Steuerschaltung, die zu Folgendem konfiguriert ist:
Antreiben der Kathode (42) und der Anode (57), um ein Schrittmachersignal an ein Herz des Patienten anzulegen,
Detektieren von zumindest einem kardiologischen Parameter unter Verwendung der zumindest zwei Erfassungs-Haut-EKG-Elektroden (106), und
zumindest teilweise als Reaktion auf den detektierten zumindest einen kardiologischen Parameter, Festlegen von Parametern des Schrittmachersignals, indem Energie von der Energieübermittlungsspule an die prothetische Klappenspule (36) durch induktive Kopplung drahtlos übertragen wird.

2. Klappenprothesensystem nach Anspruch 1, wobei die nicht implantierbare Steuerschaltung zu Folgendem konfiguriert ist:
Analysieren des detektierten zumindest einen kardiologischen Parameters, um ein Niveau an Reaktionsfähigkeit des Herzens auf das Schrittmachersignal zu bewerten, und
bei Bestimmen, dass das Niveau an Reaktionsfähigkeit unbefriedigend ist, Erhöhen einer Stärke des Schrittmachersignals als Reaktion auf den detektierten zumindest einen kardiologischen Parameter.

3. Klappenprothesensystem nach Anspruch 1,
wobei der zumindest eine kardiologische Parameter zumindest ein Zeitplanungsmerkmal beinhaltet,
wobei die Parameter des Schrittmachersignals zumindest einen Zeitplanungsparameter beinhalten, und
wobei die nicht implantierbare Steuerschaltung konfiguriert ist, um den zumindest einen Zeitplanungsparameter des Schrittmachersignals als Reaktion auf das zumindest eine Zeitplanungsmerkmal des detektierten zumindest einen kardiologischen Parameters festzulegen.

4. Klappenprothesensystem nach Anspruch 1, wobei die prothetische Aortenklappe (120) einen oder mehrere längliche isolierte elektrische Leiter (38) umfasst, welche die prothetische Klappenspule (36) direkt in der nicht drahtlosen elektrischen Kommunikation mit der Kathode (42) und der Anode (57) koppeln.

5. Klappenprothesensystem nach Anspruch 1, wobei jeweilige nicht elektrisch isolierte Endabschnitte der prothetischen Klappenspule (36) die Kathode (42) und die Anode (57) definieren.

6. Klappenprothesensystem nach Anspruch 1, wobei die nicht implantierbare Steuerschaltung konfiguriert ist, um eine Amplitude des Schrittmachersignals durch Modulieren einer Amplitude der Energie, die von der Energieübermittlungsspule an die prothetische Klappenspule (36) drahtlos übertragen wird, festzulegen.

7. Klappenprothesensystem nach Anspruch 1, wobei das Schrittmachersignal Impulse beinhaltet und wobei die nicht implantierbare Steuerschaltung konfiguriert ist, um die Kathode (42) und die Anode (57) anzutreiben, um (a) Anlegung jedes Impulses des Schrittmachersignals zu beginnen, indem damit begonnen wird, Energie von der Energieübermittlungsspule an die prothetische Klappenspule (36) drahtlos zu übertragen, und (b) die Anlegung jedes Impulses des Schrittmachersignals zu beenden, indem damit aufgehört wird, Energie von der Energieübermittlungsspule an die prothetische Klappenspule (36) drahtlos zu übertragen.

8. Klappenprothesensystem nach einem der Ansprüche 1-7, wobei die nicht implantierbare Einheit eine externe Einheit (100) ist, die konfiguriert ist, um außerhalb eines Körpers des Patienten angeordnet zu sein.

9. Klappenprothesensystem nach einem der Ansprüche 1-7,
wobei die nicht implantierbare Einheit ein Abgabesystem (70) ist, das ferner ein Abgaberohr (72) und einen oder mehrere Drähte (78) umfasst, die entlang des Abgaberohres (72) verlaufen,
wobei die Energieübermittlungsspule eine Abgabesystemspule (74) ist,
wobei die nicht implantierbare Steuerschaltung Abgabesystemsteuerschaltung (80) ist, die in elektrischer Kommunikation mit der Abgabesystemspule (74) über den einen oder die mehreren Drähte (78) ist, und
wobei die Abgabesystemspule (74) an das Abgaberohr (72) an einer distalen Stelle (76) des Abgaberohres (72) gekoppelt ist.

10. Klappenprothesensystem nach Anspruch 9, wobei die Abgabesystemsteuerschaltung (80) konfiguriert ist, um die Kathode (42) und die Anode (57) anzutreiben, um schnelles ventrikuläres Schrittmachen anzulegen, indem die Energie von der Energieübermittlungsspule drahtlos an die prothetische Klappenspule (36) durch induktive Kopplung übertragen wird.

11. Klappenprothesensystem nach einem der Ansprüche 1-7,
wobei die nicht implantierbare Steuerschaltung konfiguriert ist, um die Energie durch Erzeugen einer Vielzahl von AC-Impulsen, die jeweils eine Folge von AC-Stößen beinhalten, drahtlos zu übertragen, und
wobei die prothetische Aortenklappe (120) eine passive Diode (124) umfasst, die in elektrischer Kommunikation mit der prothetischen Klappenspule (36) gekoppelt ist und konfiguriert ist, um Strom in der prothetischen Klappenspule (36) gleichzurichten.

12. Klappenprothesensystem nach Anspruch 11, wobei die nicht implantierbare Steuerschaltung konfiguriert ist, um die Folge von AC-Stößen bei einer Frequenz zwischen 3 kHz und 130 kHz zu erzeugen.

13. Klappenprothesensystem nach Anspruch 11, wobei die nicht implantierbare Steuerschaltung konfiguriert ist, um 20-100 AC-Stöße in jedem der AC-Impulse zu beinhalten.

14. Klappenprothesensystem nach Anspruch 11, wobei die prothetische Aortenklappe (120) einen Kondensator (126) umfasst, der in elektrischer Kommunikation mit der Kathode (42) und der Anode (57) ist.

## Revendications

1. Système de prothèse valvulaire comprenant :
(i) une valvule aortique prothétique (120), qui est conçue pour être implantée dans une valvule aortique native d'un patient, et qui comprend :
(a) une pluralité de feuillets prothétiques (32) ;
(b) un cadre (30) ;
(c) une cathode (42) et une anode (57), qui sont couplées mécaniquement au cadre (30) ; et
(d) une bobine de valvule prothétique (36), qui est en communication électrique non sans fil avec la cathode (42) et l'anode (57), ladite valvule aortique prothétique (120) ne comprenant pas de composants électroniques actifs ; et
(ii) une unité non implantable, qui comprend :
(a) une bobine de transmission d'énergie ;
(b) au moins deux électrodes de détection d'ECG cutanées (106) ; et
(c) un circuit de commande non implantable, qui est conçu pour :
commander à la cathode (42) et à l'anode (57) d'appliquer un signal de stimulation sur un cœur du patient,
détecter au moins un paramètre cardiaque à l'aide desdites au moins deux électrodes de détection d'ECG cutanées (106), et
au moins partiellement en réponse audit au moins un paramètre cardiaque détecté, définir les paramètres du signal de stimulation, en transférant sans fil l'énergie de la bobine de transmission d'énergie à la bobine de valvule prothétique (36) par couplage inductif.

2. Système de prothèse valvulaire selon la revendication 1, ledit circuit de commande non implantable étant conçu pour :
analyser ledit au moins un paramètre cardiaque détecté pour évaluer un niveau de réactivité du cœur au signal de stimulation, et
après s'être assuré que le niveau de réactivité n'est pas satisfaisant, augmenter l'intensité du signal de stimulation en réponse audit au moins un paramètre cardiaque détecté.

3. Système de prothèse valvulaire selon la revendication 1,
ledit au moins un paramètre cardiaque comprenant au moins une caractéristique de synchronisation,
lesdits paramètres du signal de stimulation comprenant au moins un paramètre de synchronisation, et
ledit circuit de commande non implantable étant conçu pour définir ledit au moins un paramètre de synchronisation du signal de stimulation en réponse à ladite au moins une caractéristique de synchronisation dudit au moins un paramètre cardiaque détecté.

4. Système de prothèse valvulaire selon la revendication 1, ladite valvule aortique prothétique (120) comprenant un ou plusieurs conducteurs électriques isolés allongés (38), qui couplent directement la bobine de valvule prothétique (36) dans la communication électrique non sans fil avec la cathode (42) et l'anode (57).

5. Système de prothèse valvulaire selon la revendication 1, des parties d'extrémité respectives non isolées électriquement de la bobine de valvule prothétique (36) définissant la cathode (42) et l'anode (57).

6. Système de prothèse valvulaire selon la revendication 1, ledit circuit de commande non implantable étant conçu pour définir une amplitude du signal de stimulation en modulant l'amplitude de l'énergie transférée sans fil de la bobine de transmission d'énergie à la bobine de valvule prothétique (36).

7. Système de prothèse valvulaire selon la revendication 1, ledit signal de stimulation comprenant des impulsions, et ledit circuit de commande non implantable étant conçu pour commander à la cathode (42) et à l'anode (57) de (a) commencer l'application de chaque impulsion du signal de stimulation en commençant à transférer sans fil l'énergie de la bobine de transmission d'énergie à la bobine de valvule prothétique (36), et (b) conclure l'application de chaque impulsion du signal de stimulation en cessant de transférer sans fil l'énergie de la bobine de transmission d'énergie à la bobine de valvule prothétique (36).

8. Système de prothèse valvulaire selon l'une quelconque des revendications 1 à 7, ladite unité non implantable étant une unité externe (100), qui est conçue pour être disposée à l'extérieur du corps du patient.

9. Système de prothèse valvulaire selon l'une quelconque des revendications 1 à 7,
ladite unité non implantable étant un système d'administration (70), qui comprend en outre un tube d'administration (72), et un ou plusieurs fils (78), qui passent le long du tube d'administration (72),
ladite bobine de transmission d'énergie étant une bobine de système d'administration (74),
ledit circuit de commande non implantable étant un circuit de commande de système d'administration (80), qui est en communication électrique avec la bobine de système d'administration (74) par l'intermédiaire desdits un ou plusieurs fils (78), et
ladite bobine de système d'administration (74) étant couplée au tube d'administration (72) au niveau d'un site distal (76) du tube d'administration (72).

10. Système de prothèse valvulaire selon la revendication 9, ledit circuit de commande de système d'administration (80) étant conçu pour commander à la cathode (42) et à l'anode (57) d'appliquer une stimulation ventriculaire rapide, en transférant sans fil l'énergie de la bobine de transmission d'énergie à la bobine de valvule prothétique (36) par couplage inductif.

11. Système de prothèse valvulaire selon l'une quelconque des revendications 1 à 7,
ledit circuit de commande non implantable étant conçu pour transférer sans fil l'énergie en générant une pluralité d'impulsions de CA, chacune comprenant un train de rafales de CA, et
ladite valvule aortique prothétique (120) comprenant une diode passive (124), qui est couplée en communication électrique avec la bobine de valvule prothétique (36), et est conçue pour redresser le courant dans la bobine de valvule prothétique (36).

12. Système de prothèse valvulaire selon la revendication 11, ledit circuit de commande non implantable étant conçu pour générer le train de rafales de CA à une fréquence située entre 3 kHz et 130 kHz.

13. Système de prothèse valvulaire selon la revendication 11, ledit circuit de commande non implantable étant conçu pour comprendre de 20 à 100 rafales de CA dans chacune des impulsions de CA.

14. Système de prothèse valvulaire selon la revendication 11, ladite valvule aortique prothétique (120) comprenant un condensateur (126), qui est en communication électrique avec la cathode (42) et l'anode (57).
